Europäisches Patentamt

European Patent Office

Office européen des brevets

(11) Publication number: **0 305 900**
**A2**

## (12) EUROPEAN PATENT APPLICATION

(21) Application number: 88113875.4

(22) Date of filing: 25.08.88

(51) Int. Cl.4: **G01N 33/53 , B01L 3/00**

(30) Priority: 29.08.87 JP 215992/87

(43) Date of publication of application:
08.03.89 Bulletin 89/10

(84) Designated Contracting States:
AT BE CH DE ES FR GB GR IT LI LU NL SE

(71) Applicant: **MOCHIDA PHARMACEUTICAL CO., LTD.**
**7, Yotsuya 1-chome**
**Shinjuku-ku Tokyo 160(JP)**

(72) Inventor: **Mochida, Ei**
**2-5-4, Komagome Toshima-ku**
**Tokyo(JP)**

(74) Representative: **Füchsle, Klaus, Dipl.-Ing. et al**
**Hoffmann . Eitle & Partner Patentanwälte**
**Arabellastrasse 4**
**D-8000 München 81(DE)**

(54) Vessel for an enzymeimmunoassay.

(57) A vessel (1) for an enzymeimmunoassay comprises a dish-shaped body having a substantially flat inner bottom surface provided with at lease on depression (2; 4; 5; 6; 7; 9; 11; 12) in which an antibody or antigen can be insolubilized.

# FIG. 1A

# FIG. 1B

## VESSEL FOR AN ENZYMEIMMUNOASSAY

### BACKGROUND OF THE INVENTION

1. Field of the Invention:

This invention relates to a vessel which is used for conducting an enzymeimmunoassay.

2. Description of the Prior Art

An assay for a substance existing in a very small amount in a biological fluid is often performed for a variety of purposes including the diagnosis of disease and the examination of results of medical treatment. Various methods for such an assay have been developed and include simple methods which are easy to carry out, and sophisticated methods having a high degree of sensitivity and accuracy. An appropriate method is selected to suit the purpose for which a particular assay is intended. As the simple methods can be performed easily without the aid of any particular instrument or reaction device, they are particularly useful for the diagnosis of cases for which a qualitative or semiquantitative assay is sufficient. Therefore, they are widely used for e.g. the diagnosis of pregnancy.

There are a number of simple methods which base their principle of determination on an immunochemical reaction. These methods include an agglutination reaction method which is based on an agglutination or agglutination-inhibition reaction employing red blood cells or a latex as a carrier, and an enzymeimmunoassay (EIA) which employs an enzyme as a labeling agent.

Among the agglutination reaction methods, the method which employs red blood cells as a carrier is easy to perform and has a relatively high degree of determination sensitivity, but requires a long time for completing an assay. The method which employs a latex can be performed easily and quickly, though its sensitivity is relatively low. The latter method is, therefore, used more often than any other method for the diagnosis of cases of pregnancy, or other cases where no particularly high sensitivity is required. However, it can be used only at a hospital, or other medical organization, since a high degree of skill is required for deriving a proper conclusion from the results of an assay.

EIA is a method which enables a very high degree of sensitivity. However, it has a number of drawbacks, too. A long time of reaction is required for achieving a high degree of sensitivity. No desired sensitivity can be expected from a short time of reaction. Moreover, a complicated step is required for the B/F separation, i.e. the physical separation of the labelled antibodies which have been combined with antigens to be assayed preceding an enzyme reaction, from those which have not been combined. If said separation is not properly done, a non-specific enzyme reaction is likely to result.

The simple methods as hereinabove described, except EIA, can be used for detecting a substance, but not for determining the amount thereof, i.e. none of them is effective for quantitative determination. On the other hand, EIA is useful not only for qualitative determination, as the enzyme reaction solution presents the color developed by the substance to be assayed, if any, but also for quantitative determination, as the quantity of the substance can be estimated by the intensity of the color which it develops. The assay can be conducted easily and accurately. Despite these advantages, however, no practically useful method for an EIA has been available as yet, since all of the approaches which have hitherto been made for shortening the reaction time and simplifying the step for the B/F separation have failed to produce any satisfactory results.

### SUMMARY OF THE INVENTION

It is, therefore, an object of this invention to provide a device which can be used to facilitate an enzymeimmunoassay and, more particularly, to simplify a series of steps including the antigen-antibody reaction, the B/F separation, the enzyme reaction and the evaluation of results when an enzymeimmunoassay is conducted.

This object is essentially attained by a vessel in the shape of a dish having a substantially flat inner bottom surface provided with at least one depression to the wall of which antibodies or antigens are combined or bound, whereby the antibody or antigen is insolubilized.

Other features and advantages of this invention will become apparent from the following description and the accompanying drawings.

## BRIEF DESCRIPTION OF THE DRAWINGS

Figure 1A is a top plan view of a vessel embodying this invention;
Figure 1B is a sectional view taken along the line I-I of Figure 1A;
Figure 2A is a top plan view of a vessel embodying this invention;
Figure 2B is a sectional view taken along the line II-II of Figure 2A;
Figure 3A is a top plan view of a vessel embodying this invention.
Figure 3B is a sectional view taken along the line III-III of Figure 3A;
Figure 4A is a top plan view of a vessel embodying this invention;
Figure 4B is a sectional view taken along the line IV-IV of Figure 4A;
Figure 5A is a top plan view of a vessel embodying this invention;
Figure 5B is a sectional view taken along the line V-V of Figure 5A;
Figure 5C is a sectional view taken along the line VI-VI of Figure 5A;
Figure 6A is a top plan view of a vessel embodying this invention;
Figure 6B is a sectional view taken along the line VII-VII of Figure 6A;
Figure 7A is a top plan view of a depression formed in any such vessel;
Figure 7B is a sectional view taken along the line VIII-VIII of Figure 7A; and
Figure 8 is a fragmentary enlarged perspective view of the depression shown in Figure 7B.

## DETAILED DESCRIPTION OF THE INVENTION

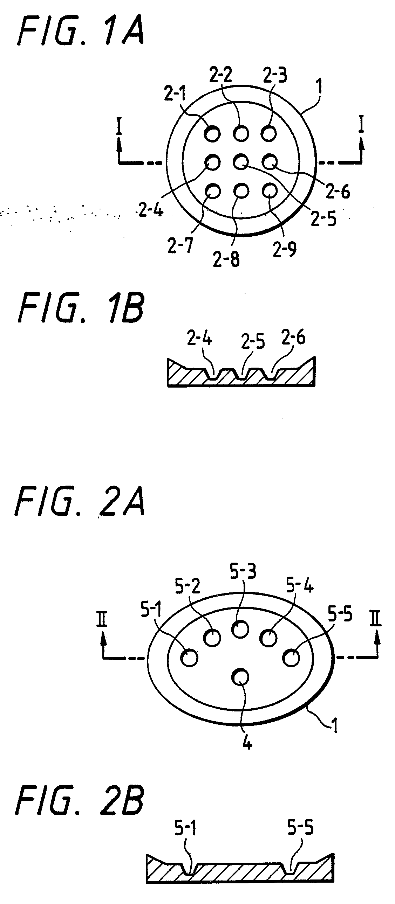

An EIA vessel embodying this invention is shown in Figures 1A and 1B. The vessel 1 is shaped like a dish and has a substantially flat inner bottom surface provided with nine depressions 2-1 to 2-9. Each depression is provided for holding an antibody or antigen which is specifically reactive with a substance to be assayed or a control substance.

While the vessel 1 shown in Figure 1A has nine depressions arranged in an array forming a matrix, the number and arrangement of the depressions depend on the purpose of an assay and the convenience of the operation involved. For example, therefore, it is beneficial to use a vessel having three depressions when a specimen, a 'positive' control substance and a 'negative' control substance are caused to undergo reactions simultaneously.

When semiquantitative determination is performed by causing a specimen and a series of standard substances diluted to different degrees to react simultaneously, and comparing the reaction of the specimen with the reactions of the standard substances, it is beneficial to use a vessel having a depression for the specimen in or about its center and a set of depressions for the standard substances arranged in an array surrounding the depression for the specimen. The depressions for the standard substances can, for example, be arranged to form a regular hexagonal array with one depression located at each corner of a regular hexagon, while the depression for the specimen is provided in the center thereof. The depressions may alternatively be arranged in a sectorial array so that the depressions for the standard substances may form an arcuate array, while the depression for the specimen is located at the center of a circle of which the sectorial array forms a part. These arrangements make it possible to compare the color produced by the specimen easily and accurately with the color produced by each standard substance and thereby improve the accuracy of semiquantitative analysis of the specimen.

It is also beneficial to arrange the depressions in an array shaped like a cross (+), so that the color produced in the depressions may form a sign looking like " + " if the specimen contains a substance to be assayed, or a sign looking like "-" if not.

The depressions are preferably so sized as to be capable of retaining a liquid in the depression when the vessel is inclined, turned upside down, or shaken lightly to allow any unnecessary amount of liquid to flow out of the depressions. More specifically, they are preferably so sized that the surface tension, or

3

capillary action of the liquid may enable it to stay in the depressions when the vessel is inclined, etc. It is, however, essential that they be sufficiently large to be easily filled with a specimen or reagent, and to be satisfactorily washed for the B/F separation. Accordingly, each depression preferably has a diameter or side length of about 2 to 5 mm.

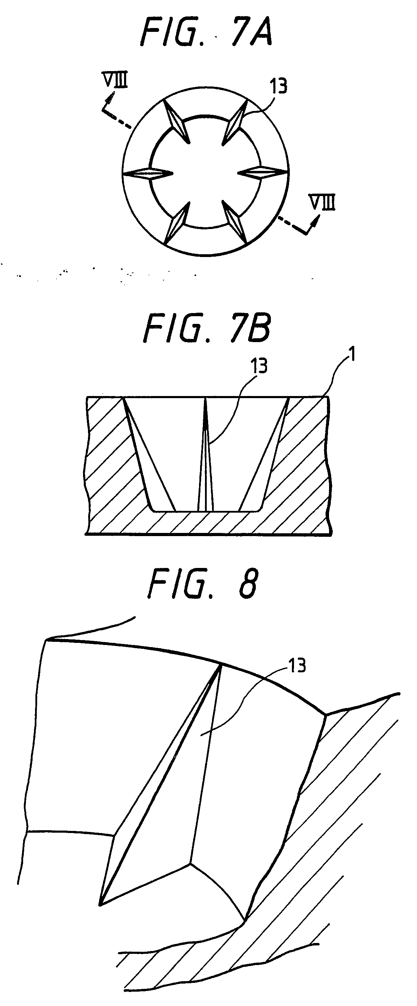

The more contact that occurs between a specimen and an insolubilized antibody or antigen, the more actively they react, and the shorter their reaction time becomes. Therefore, each depression preferably has an uneven or roughened wall surface so as to present a large surface area. This uneven surface can, for example, be obtained if the wall of the depression is provided with a plurality of pleats or ridges, as shown by way of example at 13 in Figures 7A, 7B and 8. The wall of the depression may alternatively have a fine-grained roughened surface which is similar to the surface of frosted glass.

Every two adjacent depressions are spaced apart from each other by a distance which prevents any mixing of the liquid in one depression with the liquid in the other.

The size and shape of the vessel depend on the number and spacing of the depressions. Too large a vessel should be avoided, as it requires too large amounts of a specimen and reagents which are practically disadvantageous. A circular, oval or square vessel having a diameter or side length of about 20 to 35 mm, and a rectangular vessel having a width of about 5 mm and a length of about 50 mm are, for example, appropriate, though differently sized vessels may also be appropriate in certain cases. The depressions may be circular as shown for example in Figure 1A, or of any other appropriate shape, such as square or hexagonal.

The vessel can be formed from any of various kinds of materials including glass, plastics and paper. However, it is preferably formed from plastics, and more specifically, polystyrene, polypropylene, polyacrylate or polyester. It is also desirable to employ a white material so that the color which is developed, and its intensity may be easily recognizable. The vessel can be manufactured by injection molding, extrusion, pressing, cutting, or any other method that is usually employed for manufacturing a product of a plastic material.

Any known method, such as chemical bonding or physical adsorption can be employed for binding an antibody or antigen with the wall surface of each depression to insolubilize the antibody or antigen.

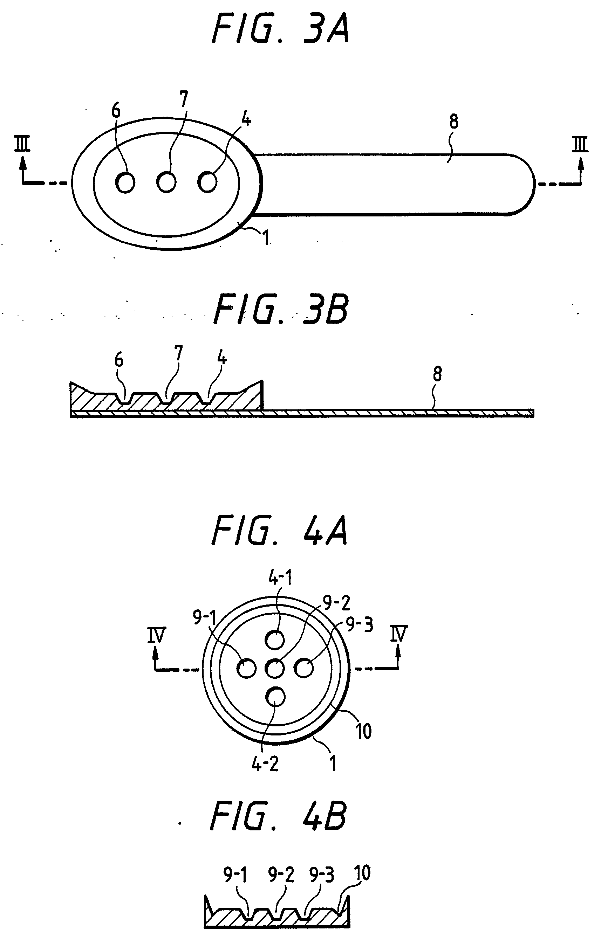

Tap water can, for example, be used to wash the vessel as a whole for the separation of bound and free antigen or antibody. The vessel is, therefore, easier to wash if it is provided with a removable handle which is attached to the vessel when it is washed, as shown by, e.g., the reference numeral 8 in Figs. 3A and 3B.

Any enzyme for labeling an antibody or antigen, any substrate acted on by the enzyme and any chromogen that has hitherto been used for an EIA can be utilized for conducting an assay by employing the vessel of this invention. The vessel may be used either for qualitative analysis, which is based on a color developed by a substance to be assayed, or for semiquantitative analysis, which is based on a comparison of the intensity of the color developed by the substance to be assayed with that of the color developed by a control substance.

Various modified forms of vessels embodying this invention are shown by way of example in Figures 2A to 6B. The vessel 1 shown in Figures 2A and 2B has a sectorial array of six depressions, i.e. a depression 4 for a specimen and five depressions 5-1 to 5-5 for differently diluted standard substances. The vessel 1 enables more accurate semiquantitative determination, as the intensity of the color developed by the specimen can be compared with the different degrees of intensity of color developed by the standard substances.

The vessel 1 shown in Figures 3A and 3B has a depression 4 for a specimen, a depression 6 for a positive control substance and a depression 7 for a negative control substance. The positive control substance comprises the substance to be assayed and the negative control substance is a substance which does not undergo any immunological cross reaction with the substance to be assayed. The vessel 1 is provided with a handle 8 which is used when the vessel is washed, as hereinbefore stated.

The vessel 1 shown in Figures 4A and 4B has a cross-shaped array of five depressions 4-1, 4-2 and 9-1 to 9-3. The depressions 4-1 and 4-2 are each provided for holding an insolubilized antibody or antigen for a substance to be assayed and the depressions 9-1 to 9-3 are each provided for holding an insolubilized antibody or antigen for a substance which is always present in a specimen, but which does not undergo any immunological cross reaction with the substance to be assayed. If the substance to be assayed is present in the specimen, a corresponding color appears in all of the depressions and forms a sign looking like " + ", but if not, the color appears in only the depressions 9-1 to 9-3 and forms a sign looking like "-". The vessel 1 also has a circumferentially extending groove 10 which facilitates draining when the depressions are washed.

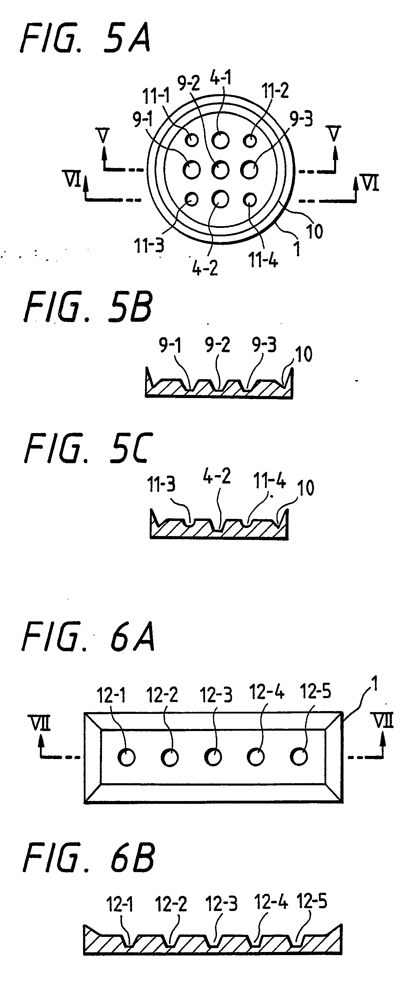

The vessel 1 shown in Figures 5A to 5C is a modified form of the vessel shown in Figures 4A and 4B. It has two depressions 4-1 and 4-2 for a specimen and three depressions 9-1 to 9-3 for a control substance which form a cross-shaped array which is identical to the array shown in Figure 4A. The vessel of Figure 5A further has four additional depressions 11-1 to 11-4 which are somewhat smaller than the other depressions and form therewith an array similar to the matrix shown in Figure 1A. The additional depressions 11-1 to 11-4 are located at the four corners, respectively, of the array and are each provided for holding an insolubilized antibody or antigen for a substance which does not undergo any immunological cross reaction with a substance to be assayed. In other words, they are each provided for ascertaining a non-specific reaction.

The vessel 1 shown in Figures 6A and 6B is rectangular and has a linear array of five depressions 12-1 to 12-5. When it is desirable to analyze a single kind of specimen containing a plurality of different substances to be assayed, up to and including five, each of the depressions can be used for holding an insolubilized antibody or antigen which reacts with only one of the substances to be assayed so that a single assay may be sufficient for simultaneously detecting all of those substances.

The use of the vessel according to this invention will now be described by way of example with reference to the qualitative and semiquantitative determination of human chorionic gonadotropin (hCG). hCG is a hormone which is produced in chorionic cells and is found in the blood and urine of a pregnant woman, a patient of a chorionic disease, etc. A qualitative assay for hCG is sufficient for diagnosis as to pregnancy, since a large amount of hCG is produced and secreted into the urine and blood of a pregnant woman. No specific color to hCG appears, if no hCG is present in the urine or blood which has been analyzed. Even if hCG is present, however, no specific color appears as a result of reaction, if the assay has not been properly conducted. In order to confirm that the lack of development of color is due to the fact that hCG is not present, therefore, it is useful to employ a simultaneous reaction for $\beta$2-microglobulin which is present in the blood and urine of any human being. The appearance of the color specific to $\beta$ 2-microglobulin confirms that the assay has been conducted correctly. The vessel shown in Figures 3A and 3B is, for example, suitable for use in the qualitative determination as hereinabove described.

When watching the post-operation course of a chorionic disease, it is necessary to determine the quantity of hCG in the urine or blood of the patient. A complicated process has hitherto been required for performing this kind of quantitative analysis. The vessel shown in Figures 2A and 2B makes it possible to carry out the semiquantitative determination of hCG easily and correctly, as the intensity of the color developed by a specimen can be compared with the intensity of the color developed by each of five different standard quantities of hCG, e.g. 0, 10, 20, 50 and 100 mIU/ml.

The use of the vessel according to this invention will now be described in further detail with reference to a number of more specific examples. In the examples which follow, "the vessel" means a vessel for an enzymeimmunoassay in accordance with the present invention.

EXAMPLE 1 - Use of the Vessel for an Allergen Assay:

The vessel shown in Figures 1A and 1B was employed. The antigens which had been extracted from house dust, Dermatophagoides pteronysinus, Dermatophagoides farinae, Aspergillus fumigatus, Candida albicans, Japanese cedar pollen, ragweed pollen of a ragweed, egg white and milk, respectively, were each dissolved in a 0.05M phosphate buffered saline solution (PBS)/(pH 6.4) to prepare a solution with a concentration of 100 μg/ml. The solutions each containing one of the antigens were put in the nine depressions 2-1 to 2-9, respectively. The vessel was left at 37° C for an hour and was then washed with PBS, whereby the antigens were insolubilized in the depressions.

Each depression was then supplied with 10 μl of the serum of a patient having an allergic disease and 5 μl of a dilute solution of an anti-IgE antibody labeled with horseradish peroxidase (enzyme). They were allowed to react together for 10 minutes and the vessel was then washed with PBS. Then, each depression was supplied with 5 μl of each of an aqueous solution of hydrogen peroxide and a solution of tetramethylbenzidine. After five minutes, a blue color indicating a positive reaction appeared in various depressions in the vessel for the serum of some of the 10 patients for which an allergen assay had been conducted. One of the patients showed a positive reaction with house dust, another with house dust and Dermatophagoides pteronysinus, two other patients with Dermatophagoides pteronysinus, and four others with Japanese cedar pollen, while neither of the other two patients showed any positive reaction with any of the nine kinds of antigens.

Prior to this invention, an allergen assay had been carried out by a patch test conducted by injecting an antigen into the skin of a patient, a RAST employing a radioactive isotope, etc. All of those methods had, however, been complicated. The vessel of this invention was found to be capable of simplifying any such assay.

EXAMPLE 2 - Use of the Vessel for a Pregnancy Test:

The vessel shown in Figures 5A to 5C was employed. Each of two depressions 4-1 and 4-2 was supplied with a solution of an anti-hCG antibody diluted with PBS, each of three depressions 9-1 to 9-3 with a solution of an anti-$\beta$2-microglobulin antibody diluted with PBS, and each of the remaining four depressions 11-1 to 11-4 with a solution of IgG diluted with PBS, each solution having a concentration of 100 $\mu$g/ml. The vessel was left to stand at a temperature of 37°C for an hour and was then washed with PBS. Then, the vessel was supplied with 1 ml of a solution which had been obtained by diluting a mixture of an enzyme-labeled anti-hCG antibody and an enzyme-labeled anti-$\beta$2-microglobulin antibody with PBS, and was subjected to freeze drying.

The vessel was used for analyzing the urine of each of 10 pregnant women and 10 non-pregnant women. The vessel was supplied with 1 ml of the urine and after two minutes of reaction, it was washed with tap water for 10 seconds. Then, each depression was supplied with 5 $\mu$l of each of an aqueous solution of hydrogen peroxide and a solution of tetramethylbenzidine and after one minute, its color was examined. An intense blue color was observed in all of the depressions 4-1, 4-2 and 9-1 to 9-3 in each of the vessels which had been used for analyzing the urine of the 10 pregnant women, and confirmed that they were pregnant. The colored depressions formed a sign looking like " + ". On the other hand, each of the vessels which had been used for analyzing the urine of the 10 non-pregnant women developed an intense blue color only in the depressions 9-1 to 9-3, whereby a sign looking like "-" was formed. This confirmed that they were not pregnant. The signs " + " and "-" were useful for distinguishing pregnant women from non-pregnant ones easily. No such color was developed in any of the depressions 11-1 to 11-4 of any vessel.

A similar test was conducted by repeating the foregoing procedures, except that each vessel was washed with tap water for only five seconds. A blue color was developed in each of the depressions 11-1 to 11-4, too, though its intensity was lower. The washing time of five seconds was, therefore, found insufficient.

The vessel of this invention, thus, enables a very accurate pregnancy test by analyzing the urine for both hCG, which is present only in the urine of a pregnant woman, and $\beta$2-microglobulin, which is present in the urine of any person, whether she may be pregnant or not. Moreover, the test is easy to perform even by an unskilled person, as the vessel makes it possible to distinguish a non-specific reaction easily from a specific one.

EXAMPLE 3 - Use of the Vessel for a Tumor Marker Test:

The vessel shown in Figures 6A and 6B was employed. Each of the five depressions 12-1 to 12-5 was supplied with a solution obtained by diluting an antibody against CEA (carcino-embryonic antigen), AFP ($\alpha$-fetoprotein), hCG, SCC (squamous cell carcinoma related antigen) and ferritin, respectively, with PBS and having a concentration of 10 $\mu$g/ml. The vessel was left at 38°C for an hour and was then washed with PBS.

A tumor marker test was conducted with the serum of 10 patients having colorectal cancer, 10 patients having hepatoma, 10 patients having lung cancer, 10 patients having a chorionic disease, and 10 normal persons, while the negative-positive cut-off values of CEA, AFP, hCG, SCC and ferritin were selected at 5 ng/ml, 20 ng/ml, 5 mIU/ml, 2 ng/ml and 100 ng/ml, respectively.

The depressions of each of the vessels which had been prepared for analyzing the serum of each of those persons were each supplied with 10 $\mu$l of the serum of the same person. They were also supplied with 5 $\mu$l of a solution of an enzyme-labeled antibody which had been diluted to attain the corresponding cut-off value as hereinabove stated. After 10 minutes of reaction, the vessel was washed with PBS. Then, each depression was supplied with 5 $\mu$l of each of an aqueous solution of hydrogen peroxide and a solution of o-phenylenediamine. Each of the depressions in which a yellowish brown color appeared after two minutes was concluded as one in which a positive reaction had taken place.

Six of the 10 patients having colorectal cancer showed a positive reaction with CEA, two with ferritin;

eight of the 10 hepatoma patients tested positive with AFP, five with ferritin; three of the 10 lung cancer patients tested positive with CEA, and three with SCC antigen, while all of the 10 patients having a chorionic disease showed a positive reaction with hCG.

While prior to this invention, it had been necessary to perform quantitative analysis with each antigen individually, the vessel of this invention was found to enable an assay for a plurality of tumor markers all at once and to be, therefore, useful for a screening test.

As the vessel of this invention has a substantially flat inner bottom surface provided with one or more depressions in which an antibody or antigen is insolubilized, it is possible to supply a specimen, a reagent or a washing solution into all of the depressions simultaneously. It is not necessary to supply a specimen, etc. into each depression individually, or wash each depression individually for the separation of bound and free antigen or antibody. This quickens and simplifies an assay. However, it is, of course, possible to supply a specimen of reagent into each depression individually.

The vessel is small and each depression is, of course, much smaller. Therefore, the vessel calls for only a small amount of a specimen or reagent, or an antibody or antigen to be insolubilized. As each depression is small, it has a large surface area relative to its volume. This feature contributes to promoting the reaction between an insolubilized antibody or antigen and a specimen, or between an enzyme and a substrate, thereby greatly shortening the time required for an assay.

Table 1 compares an assay for hCG conducted by using the vessel of this invention as shown in Figures 2A and 2B, with the assays conducted by the conventional EIA and also by the conventional method employing hemagglutination.

TABLE 1

|  | Method with the vessel of the Invention | Conventional EIA Method | Hemagglutination Method |
| --- | --- | --- | --- |
| Sensitivity of analysis | 5 mIU/ml | 5 mIU/ml | 5 mIU/ml |
| Number of measurements | 1 | 1 | 5 |
| Time required | 5 min. | 30 min.- 4 hr. | 2 hr. |
| Amount of specimen required | 10 μl | 50 to 200 μl | 100 μl |
| Analysis | Semiquantitative | Quantitative | Semiquantitative |
| Other instrument | Not required | Required | Not required |

As is obvious from Table 1, the same sensitivity was employed for comparing the three methods and the use of the vessel according to this invention enabled a quick and simple assay by achieving a great reduction in the time and the amount of the specimen required, and without calling for any other instrument, though it was a semiquantitative assay.

The foregoing describes the cases wherein an antibody or antigen is used as a substance which is combined or bound to the depressions of the vessel. The present invention is also directed to cases wherein use is made of substances other than an antibody or antigen which substances are specifically bindable to a substance to be measured. Accordingly, various kinds of assays other than the EIA can be conducted using the vessel of the present invention.

## Claims

1. A vessel (1) for an enzymeimmunoassay comprising a dish-shaped body having a substantially flat inner bottom surface provided with at lease one depression (2; 4; 5; 6; 7; 9; 11; 12) having a wall which is adapted to adhere to an antibody or antigen to insolubilize said antigen or antibody.

2. A vessel as set forth in claim 1, wherein said depression (2; 4; 5; 6; 7; 9; 11; 12) has an uneven wall surface.

3. A vessel as set forth in claim 1, wherein said bottom surface is provided with a plurality of depressions (2; 4; 5; 6; 7; 9; 11; 12) arranged in a matrix.

4. A vessel as set forth in claim 1, wherein said bottom surface is provided with a plurality of depressions (2; 4; 5; 6; 7; 9; 11; 12) forming a sectorial array.

5. A vessel as set forth in claim 1,
wherein said bottom surface is provided with a plurality of depressions (2; 4; 5; 6; 7; 9; 11; 12) forming a cross-spaced array.

6. A vessel as set forth in claim 1,
wherein said bottom surface is provided with a plurality of depressions (2; 4; 5; 6; 7; 9; 11; 12) forming a linear array.

7. A vessel as set forth in claim 1, further including a handle (8) attached to said body.

8. A vessel as set forth in claim 2,
wherein said uneven wall surface is defined by a plurality of ridges (13).

9. A vessel as set forth in claim 1,
wherein said body has a circular, oval, square, hexagonal or rectangular shape.

10. A vessel as set forth in claim 1,
wherein said bottom surface is provided with a plurality of differently sized depressions (2; 4; 5; 6; 7; 9; 11; 12).

11. A vessel (1) for use in determining the amount of a test material comprising a dish-shaped body having a substantially flat inner bottom surface provided with at least one depression (2; 4; 5; 6; 7; 9; 11; 12) having a wall which is adapted to adhere to a substance to insolubilize said substance, said substance being specifically bindable to a test material.

12. A vessel as set forth in claim 11,
wherein said depression (2; 4; 5; 6; 7; 9; 11; 12) has an uneven wall surface.

13. A vessel as set forth in claim 11,
wherein said bottom surface is provided with a plurality of depressions (2; 4; 5; 6; 7; 9; 11; 12) arranged in a matrix.

14. A vessel as set forth in claim 11,
wherein said bottom surface is provided with a plurality of depressions (2; 4; 5; 6; 7; 9; 11; 12) forming a sectorial array.

15. A vessel as set forth in claim 11,
wherein said bottom surface is provided with a plurality of depressions (2; 4; 5; 6; 7; 9; 11; 12) forming a cross-spaced array.

16. A vessel as set forth in claim 11,
wherein said bottom surface is provided with a plurality of depressions (2; 4; 5; 6; 7; 9; 11; 12) forming a linear array.

17. A vessel as set forth in claim 11,
further including a handle (8) attached to said body.

18. A vessel as set forth in claim 12,
wherein said uneven wall surface is defined by a plurality of ridges (13).

19. A vessel as set forth in claim 11,
wherein said body has a circular, oval, square, hexagonal or rectangular shape.

20. A vessel as set forth in claim 11,
wherein said bottom surface is provided with a plurality of differently sized depressions (2; 4; 5; 6; 7; 9; 11; 12).

FIG. 1A

FIG. 1B

FIG. 2A

FIG. 2B

# FIG. 3A

# FIG. 3B

# FIG. 4A

# FIG. 4B

FIG. 5A

FIG. 5B

FIG. 5C

FIG. 6A

FIG. 6B

FIG. 7A

FIG. 7B

FIG. 8